# EUROPÄISCHE PATENTSCHRIFT

(11) **EP 3 578 956 B1**
(45) Veröffentlichungstag und Bekanntmachung des Hinweises auf die Patenterteilung: **13.01.2021**
(21) Anmeldenummer: 19178398.4
(22) Anmeldetag: 05.06.2019
(51) Int. Cl.: G01N 21/33, G01N 21/84, G01N 21/85, C12Q 1/30, G01N 21/53

(54) **VERFAHREN UND MESSVORRICHTUNG ZUM BESTIMMEN EINER PERESSIGSÄURE-KONZENTRATION IN EINEM PERESSIGSÄURE- UND WASSERSTOFFPEROXIDHALTIGEN STERILISIERUNGSMEDIUM**
METHOD AND MEASURING DEVICE FOR DETERMINING A PERACETIC ACID CONCENTRATION IN A STERILISATION MEDIUM CONTAINING PERACETIC ACID AND HYDROGEN PEROXIDE
PROCÉDÉ ET DISPOSITIF DE MESURE PERMETTANT DE DÉTERMINER UNE CONCENTRATION D'ACIDE PERACÉTIQUE DANS UN MILIEU DE STÉRILISATION CONTENANT DE L'ACIDE PERACÉTIQUE ET DU PEROXYDE D'HYDROGÈNE

(30) Priorität: 05.06.2018 DE 102018113300
(43) Veröffentlichungstag der Anmeldung: 11.12.2019
(73) Patentinhaber: KRONES AG, 93073 Neutraubling (DE)
(72) Erfinder: Soellner, Juergen, 93073 Neutraubling (DE); Dirscherl, Armin, 93073 Neutraubling (DE)
(74) Vertreter: Nordmeyer, Philipp Werner

(56) Entgegenhaltungen:
- EP-A1- 2 880 977
- JP-A- H0 445 798
- US-A1- 2010 206 787
- US-A1- 2018 024 103

## Beschreibung

### Technisches Gebiet

Die vorliegende Erfindung betrifft ein Verfahren und eine Messvorrichtung zum Bestimmen einer Peressigsäure-Konzentration in einem peressigsäure- und wasserstoffperoxidhaltigen Sterilisierungsmedium zur Verwendung in einer Getränkeabfüllanlage. Ferner betrifft die vorliegende Erfindung eine Getränkeabfüllanlage, die eine derartige Messvorrichtung umfasst.

### Stand der Technik

Zur Einhaltung von Hygieneanforderungen werden bei der Verpackung von Lebensmitteln aseptische Anlagen eingesetzt, in denen Prozessgüter unter sterilen Bedingungen verarbeitet werden. Hierzu zählen beispielsweise Getränkeabfüllanlagen, in denen flüssige Füllprodukte abgefüllt werden.

Derartige Anlagen weisen von der Umgebung abgegrenzte Reinräume auf, die vom Füllprodukt selbst sowie von den Packmitteln, insbesondere von zu befüllenden Behältern durchlaufen werden und in denen Verarbeitungsstationen der Anlage, beispielsweise in Form von Streckblasvorrichtungen zum Herstellen der zu befüllenden Behälter, Rinsevorrichtungen zum Rinsen der zu befüllenden Behälter, Füllvorrichtungen zu Befüllen der zu befüllenden Behälter mit dem Füllprodukt, oder Verschließvorrichtungen zum Verschließen der mit dem Füllprodukt befüllten Behälter angeordnet sind. Die Anlagen können zur Sicherstellung von sterilen Bedingungen turnusgemäß oder anlassbezogen einer Sterilisierung beispielsweise unter Einsatz eines Sterilisierungsmediums zur chemischen Entkeimung unterzogen werden.

Auf dem Gebiet von Getränkeabfüllanlagen ist es bekannt, wasserstoffperoxidhaltige Reinigungslösungen als Sterilisierungsmedium für in der Anlage zu befüllende Behältnisse, zum Beispiel PET-Flaschen, Dosen oder Glasflaschen, zu verwenden.

Ferner ist der Einsatz von peressigsäurehaltigen Reinigungslösungen als Sterilisierungsmedium in Getränkeabfüllanlagen bekannt, wie zum Beispiel aus der DE 10 2013 100 284 A1. Peressigsäurehaltige Reinigungslösungen haben die Eigenschaft, dass sich in diesen ein Gleichgewicht zwischen Peressigsäure, Essigsäure, Wasserstoffperoxid und Wasser einstellt.

Zur Wiederverwertung des Sterilisierungsmediums sind Reinigungssysteme für Getränkeabfüllanlagen bekannt, die einen Kreislauf für das Sterilisierungsmedium aufweisen, in dem bereits zur Sterilisation verwendetes Sterilisierungsmedium gesammelt, aufbereitet und darauffolgend erneut zur Sterilisierung verwendet wird. Bei der Verwendung von peressigsäurehaltigen Reinigungslösungen als Sterilisierungsmedium kann sich in derartigen Reinigungssystem nach Versprühen der Reinigungslösung in den Reinräumen, d.h. nach erfolgter Sterilisation, ein neues Gleichgewicht in der Reinigungslösung zwischen Peressigsäure, Essigsäure, Wasserstoffperoxid und Wasser einstellen. Dies kann beispielsweise durch Verwässerung oder durch Reaktion der darin enthaltenden Oxidationsmittel beim Sterilisieren erfolgen.

Der durch das Sterilisierungsmedium zu erzielende Reinigungseffekt hängt jedoch wesentlich von der chemischen Zusammensetzung und den Anteilen der Oxidationsmittel in der Reinigungslösung ab. Zur Wiederverwertung und entsprechend zur Aufbereitung der gesammelten und bereits zur Sterilisation verwendeten Reinigungslösung besteht somit Bedarf nach einem Messverfahren oder einer Messvorrichtung, die ein Bestimmen und eine Konzentrationsüberwachung von Anteilen der in der Reinigungslösung enthaltenen Oxidationsmittel, insbesondere der Peressigsäure, ermöglichen.

Aus der US 2010/0206787 A1 ist ein optisches Messverfahren bekannt, bei dem eine Extinktion von UV-Strahlung, d.h. eine Abschwächung der UV-Strahlung, beim Durchqueren einer peressigsäure- und wasserstoffperoxidhaltigen Lösung gemessen wird. Dem Verfahren liegt die Erkenntnis zugrunde, dass die gemessene Extinktion mit der summierten Konzentration von Peressigsäure und Wasserstoffperoxid in der Lösung korreliert. Entsprechend ermöglicht das Verfahren auf der Grundlage der gemessenen Extinktion die summierte Menge von Peressigsäure und Wasserstoffperoxid in der Lösung zu bestimmen.

Aus dem Stand der Technik sind weiterhin elektrolytische Messverfahren, wie zum Beispiel aus der EP 0 581 081 A1, und Titrationsmessverfahren, wie zum Beispiel aus der EP 2 880 977 A1, bekannt. Gegenüber den bekannten optischen Messverfahren ermöglichen diese eine Konzentration von Peressigsäure alleine und nicht summiert mit Wasserstoffperoxid in einer peressigsäure- und wasserstoffperoxidhaltigen Lösung zu bestimmen.

Die elektrolytischen Messverfahren und die Titrationsmessverfahren lassen eine Bestimmung nur der Peressigsäure-Konzentration nur mit einer zeitlichen Verzögerung zu.

Die US 2018/0024103 A1 beschreibt ein Verfahren zur Bestimmung einer Peressigsäure-Konzentration in einem Sterilisierungsmedium, das Peressigsäure und Wasserstoffperoxid enthält. Dabei wird das Wasserstoffperoxid durch Zugabe eines Katalase-Enzyms zu Sauerstoff und Wasser umgesetzt und der Gehalt des entstehenden Sauerstoffs mittels eines Lumineszenzsensors gemessen. Die JP H04 45798 A betrifft die fraktionierte Bestimmung von Wasserstoffperoxid und Peressigsäure.

### Darstellung der Erfindung

Ausgehend von dem bekannten Stand der Technik ist es eine Aufgabe der vorliegenden Erfindung, ein verbessertes Verfahren zum Bestimmen einer Peressigsäure-Konzentration in einem peressigsäure- und wasserstoffperoxidhaltigen Sterilisierungsmedium in einer Getränkeabfüllanlage bereitzustellen.

Die Aufgabe wird durch ein Verfahren mit den Merkmalen des Anspruchs 1, mit einer Messvorrichtung mit den Merkmalen des Anspruchs 4 und mit einer Getränkeabfüllanlage, umfassend eine derartige Messvorrichtung, mit den Merkmalen des Anspruchs 12 gelöst. Vorteilhafte Weiterbildungen ergeben sich aus den Unteransprüchen sowie der vorliegenden Beschreibung und den Figuren.

Entsprechend wird ein Verfahren zum Bestimmen einer Peressigsäure-Konzentration in einem peressigsäure- und wasserstoffperoxidhaltigen Sterilisierungsmedium in einer Getränkeabfüllanlage vorgeschlagen. Das Verfahren umfasst einen Schritt des Behandelns des Sterilisierungsmediums zur Umsetzung von in dem Sterilisierungsmedium enthaltenem Wasserstoffperoxid zu Sauerstoff und Wasser und einen Schritt des Bestimmens der Peressigsäure-Konzentration in dem behandelten Sterilisierungsmedium mittels eines optischen Messverfahrens.

Die vorliegende Erfindung hat erkannt, dass für die Anwendung in Getränkeabfüllanlagen optische Messverfahren gegenüber den aus dem Stand der Technik bekannten elektrolytischen Messverfahren oder Titrationsmessverfahren zur Analyse eines Sterilisierungsmediums mehrere Vorteile aufweisen. Optische Messverfahren ermöglichen zum einen eine Analyse des zu untersuchenden Sterilisierungsmediums nahezu ohne zeitliche Verzögerung, wodurch insbesondere eine Inline-Messung oder Online-Messung ermöglicht wird. Auf diese Weise kann überwacht werden, ob das für die Sterilisation verwendete Sterilisationsmedium eine hierfür vorgesehene Konzentration an Peressigsäure aufweist.

Des Weiteren sind optische Messverfahren weniger durchfluss- und temperaturabhängig und verlangen einen geringeren Implementierungs-und Instandhaltungsaufwand. Ein für die Anwendung in Getränkeabfüllanlagen weiterer Vorteil besteht darin, dass ein optisches Messverfahren aufwandsreduziert unter sterilen Bedingungen erfolgen kann, da hier ein berührungsloses Messen erfolgt.

In den vorbekannten optischen Messverfahren wird eine Extinktion von durch eine zu analysierende Lösung tretender UV-Strahlung gemessen. Derartigen Messverfahren liegt die Erkenntnis zugrunde, dass die gemessene Extinktion im Wesentlichen linear mit der Menge der in der Lösung vorhandenen Oxidationsmittel, wie zum Beispiel Peressigsäure und/oder Wasserstoffperoxid, korreliert. Die unterschiedlichen Oxidationsmittel beeinflussen die durch die Lösung tretende UV-Strahlung dabei im Wesentlichen auf gleiche Weise. Entsprechend kann durch diese Verfahren nicht zwischen den unterschiedlichen, in derselben Lösung enthaltenen Oxidationsmitteln unterschieden werden. Enthält jedoch die zu analysierende Lösung sowohl Peressigsäure als auch Wasserstoffperoxid, kann mit den bekannten Verfahren zwar eine summierte Konzentration von Peressigsäure und Wasserstoffperoxid bestimmt werden, jedoch nicht eine Konzentration von Peressigsäure alleine.

Indem das vorgeschlagene Verfahren einen Schritt des Behandelns des Sterilisierungsmediums zur Umsetzung von in dem Sterilisierungsmedium enthaltenem Wasserstoffperoxid zu Sauerstoff und Wasser umfasst, erfolgt zunächst vor Durchführung eines optischen Messverfahrens ein Abbau des in dem Sterilisierungsmedium enthaltenen Wasserstoffperoxids.

Der Schritt des Behandelns des Sterilisierungsmediums zur Umsetzung von in dem Sterilisierungsmedium enthaltenem Wasserstoffperoxid zu Sauerstoff und Wasser kann durch Zuführen eines Katalase-Enzyms erreicht werden. Genauer bewirkt das Katalase-Enzym die Umsetzung des Wasserstoffperoxids zu Sauerstoff und Wasser. Auf diese Weise kann aus dem behandelten Sterilisierungsmedium Wasserstoffperoxid vollständig entfernt werden. In dem nächsten Schritt kann dann die Peressigsäure-Konzentration in dem behandelten Sterilisierungsmedium mittels des optischen Messverfahrens bestimmt werden.

Mit anderen Worten, durch Zuführen des Katalase-Enzyms in das Sterilisierungsmedium kann in dem vorgeschlagenen Verfahren sichergestellt werden, dass zum Zeitpunkt des Durchführens des optischen Messverfahrens im Wesentlichen kein Wasserstoffperoxid in dem Sterilisierungsmedium vorhanden ist. Auf diese Weise kann durch das vorgeschlagene Verfahren die Konzentration von Peressigsäure in dem Sterilisierungsmedium gemessen werden, wodurch eine genaue Analyse ermöglicht wird. Im Ergebnis wird so ein Verfahren bereitgestellt, dass die voranstehend genannten Vorteile eines optischen Messverfahrens aufweist und gleichzeitig eine genaue Analyse des zu untersuchenden Sterilisierungsmediums ermöglicht.

Das vorgeschlagene Verfahren wird vorzugsweise in Getränkeabfüllanlagen angewendet, kann jedoch in beliebigen aseptischen Anlagen oder anderen Anlagen zum Bestimmen einer Peressigsäure-Konzentration in einem peressigsäure- und wasserstoffperoxidhaltigen Sterilisierungsmedium zum Einsatz kommen. Das peressigsäure- und wasserstoffperoxidhaltige Sterilisierungsmedium ist vorzugsweise in Form einer Lösung, d.h. in flüssiger Form bereitgestellt.

In dem Schritt des Bestimmens der Peressigsäure-Konzentration kann ein optisches Messverfahren zum Einsatz kommen, bei dem eine Extinktion einer das behandelte Sterilisierungsmedium durchsetzenden Strahlung gemessen wird und die Peressigsäure-Konzentration in Abhängigkeit der gemessenen Extinktion bestimmt wird. Die das behandelte Sterilisierungsmedium durchsetzende Strahlung ist vorzugsweise eine Strahlung im ultravioletten Wellenlängenbereich, d.h. UV-Strahlung. Die UV-Strahlung kann beispielsweise einen Wellenlängenbereich zwischen 200 nm und 300 nm aufweisen.

Dem optischen Messverfahren kann ein mathematisches Modell zugrunde liegen, das eine Korrelation zwischen der Peressigsäure-Konzentration und einer Extinktion von das Sterilisierungsmedium durchsetzender UV-Strahlung abbildet. Diese Korrelation kann im Wesentlichen eine lineare bzw. proportionale Korrelation zwischen der Peressigsäure-Konzentration und der Extinktion der UV-Strahlung sein. Das mathematische Modell kann dazu genutzt werden, die Peressigsäure-Konzentration als Funktion der Extinktion der UV-Strahlung zu definieren. Entsprechend wird durch das Verfahren ermöglicht, die Peressigsäure-Konzentration in Abhängigkeit der Extinktion der UV-Strahlung zu bestimmen. Der grundlegende Ablauf zum Bestimmen des dem optischen Messverfahren zugrunde liegenden mathematischen Modells, beispielsweise auf der Grundlage von Referenzversuchen, ist dem Fachmann aus dem Stand der Technik bekannt und wird hier somit nicht näher erläutert.

In dem vorliegenden Verfahren wird die katalytische Wirkung von Katalase-Enzymen auf Wasserstoffperoxid genutzt, wodurch Wasserstoffperoxid zu Sauerstoff und Wasser umgesetzt wird. Entsprechend wird durch Zuführen des Katalase-Enzyms in dem vorliegenden Verfahren ein Abbau von Wasserstoffperoxid in dem Sterilisierungsmedium initiiert. Das Katalase-Enzym ist vorzugsweise ein gegenüber Säuren beständiges Katalase-Enzym. Mit anderen Worten, das Katalase-Enzym ist vorzugsweise derart gewählt, dass seine katalytische Wirkung auf Wasserstoffperoxid, d.h. die katalysierte Reaktion mit Wasserstoffperoxid, bei einem in dem Sterilisierungsmedium vorherrschenden sauren pH-Bereich, beispielsweise in einem pH-Bereich zwischen 2,6 bis 6, entfaltet. Beispielsweise kann das Katalase-Enzym ein von Bakterien oder Pilzen abstammendes Enzym sein. Vorzugsweise ist das Katalase-Enzym ein von dem Pilz "Aspergillus niger" abstammendes Enzym. Für die Anwendung in aseptischen Anlagen geeignete Katalase-Enzyme sind in der WO 2009/128049 A2, Seite 4, Zeile 18 bis Seite 7, Zeile 10, offenbart, wie zum Beispiel "Genencor CA-100", "Genencor CA-400", "Mitsubishi Gas and Chemical (MGC) ASC super G" und "MGC ASC super 200".

In dem Verfahrensschritt des Behandelns des Sterilisierungsmediums kann ein Katalase-Enzym eines bestimmten Typs oder unterschiedlicher Typen verwendet werden. Das Katalase-Enzym kann in unterschiedlicher Form dem Sterilisierungsmedium zugeführt werden, zum Beispiel in Form einer das Katalase-Enzym enthaltenden Lösung, in Pulverform, etc. Die Menge an dem Sterilisierungsmedium zugeführten Katalase-Enzym ist vorzugsweise derart gewählt, dass eine vollständige Umsetzung des in dem Sterilisierungsmedium enthaltenen Wasserstoffperoxid in einer vorgegebenen Zeit sichergestellt werden kann. Beispielsweise kann in dem Schritt des Behandelns des Sterilisierungsmediums das Katalase-Enzym in einer Menge von 0.1 µg/ml bis 30 µg/ml oder mehr als 20 µg/ml, bezogen auf das Sterilisierungsmedium zugeführt werden.

Um eine genaue Bestimmung der Peressigsäure-Konzentration in dem behandelten Sterilisierungsmedium zu ermöglichen, erfolgt der Schritt des Bestimmens der Peressigsäure-Konzentration vorzugsweise nachdem das in dem Sterilisierungsmedium enthaltene Wasserstoffperoxid vollständig mittels des Katalase-Enzyms umgesetzt, d.h. vollständig abgebaut wurde. Auf diese Weise kann sichergestellt werden, dass die in dem Schritt des Bestimmens der Peressigsäure-Konzentration gemessene Extinktion nicht von in dem Sterilisierungsmedium vorhandenem Wasserstoffperoxid beeinflusst wird.

Die Menge an durch das Katalase-Enzym umgesetztem Wasserstoffperoxid hängt dabei im Wesentlichen von der seit dem Zuführen des Katalase-Enzyms verstrichenen Zeit ab. Um eine vollständige Umsetzung von Wasserstoffperoxid sicherzustellen, kann das Verfahren derart ausgeführt werden, dass zwischen dem Schritt des Behandelns des Sterilisierungsmediums und des Bestimmens der Peressigsäure-Konzentration eine Verweildauer abgewartet wird. Die Verweildauer ist vorzugsweise derart gewählt, dass nach Ablauf dieser ein Reaktionsende der durch das Katalase-Enzym hervorgerufenen Umsetzung von Wasserstoffperoxid erreicht wird. Mit anderen Worten, ein Reaktionsende der durch das Katalase-Enzym hervorgerufenen Umsetzung von Wasserstoffperoxid ist dann erreicht, wenn das Wasserstoffperoxid in dem behandelten Sterilisierungsmedium vollständig abgebaut ist.

Die Verweildauer kann vorbestimmt bzw. vorgegeben sein. Mit anderen Worten, in dem Verfahren kann der Schritt des Bestimmens der Peressigsäure-Konzentration nach einer vorgegebenen Verweildauer nach dem Schritt des Behandelns des Sterilisierungsmediums erfolgen, nach der ein Reaktionsende der durch das Katalase-Enzym hervorgerufenen Umsetzung von Wasserstoffperoxid erreicht wird. Die Verweildauer kann beispielsweise auf der Grundlage von Referenzversuchen festgelegt werden. Zum Festlegen der Verweildauer kann insbesondere die Menge und die Art des dem Sterilisierungsmedium zugeführten Katalase-Enzyms berücksichtigt werden. Dabei kann mit zunehmender Menge des dem Sterilisierungsmedium zugeführten Katalase-Enzyms die festgelegte Verweildauer geringer werden. Die Verweildauer wird vorzugsweise in Abhängigkeit der Art, der Menge, der Temperatur und/oder der Konzentration des Katalase-Enzyms bestimmt. Beispielsweise kann die Verweildauer zwischen 20 Sekunden und 30 Sekunden betragen.

Erfindungsgemäß umfasst das Verfahren einen Schritt des Ermittelns des Reaktionsendes der durch das Katalase-Enzym hervorgerufenen Umsetzung von Wasserstoffperoxid. Insbesondere kann in dem Verfahrensschritt bestimmt werden, ob zu einem Zeitpunkt, in dem der Verfahrensschritt ausgeführt wird, ein Reaktionsende vorliegt oder nicht. Der Schritt des Bestimmens der Peressigsäure-Konzentration kann entsprechend in Abhängigkeit des Schritts zum Ermitteln des Reaktionsendes der durch das Katalase-Enzym hervorgerufenen Umsetzung von Wasserstoffperoxid in dem Sterilisierungsmedium erfolgen. Beispielsweise kann der Schritt des Bestimmens der Peressigsäure-Konzentration in dem behandelten Sterilisierungsmedium erfolgen, nachdem ein Reaktionsende der durch das Katalase-Enzym hervorgerufenen Umsetzung bestimmt wurde. Mit anderen Worten, das Verfahren kann derart ausgeführt werden, dass sobald ein Reaktionsende bestimmt wird, darauffolgend der Schritt des Bestimmens der Peressigsäure-Konzentration durchgeführt wird.

Bei der durch das Katalase-Enzym hervorgerufenen Umsetzung bzw. Zersetzung des Wasserstoffperoxids in dem Sterilisierungsmedium entsteht Sauerstoff, der in Form von Gasbläschen innerhalb des vorzugsweise flüssigen Sterilisierungsmediums freigegeben wird. Die so freigesetzten Sauerstoffbläschen bewirken eine Trübung des behandelten Sterilisierungsmediums, welche das optische Messverfahren beeinflusst.

Aufbauend darauf wurde bei dem hier vorgeschlagenen Verfahren erkannt, dass sich die durch die Umsetzung von Wasserstoffperoxid hervorgerufene Trübung in dem Sterilisierungsmedium als Indikator dafür eignet, ob ein Reaktionsende der Umsetzung des Wasserstoffperoxids erreicht ist oder der Umsetzungsvorgang anhält. In dem Verfahren gemäß der Erfindung erfolgt somit in dem Schritt des Ermittelns des Reaktionsendes der durch das Katalase-Enzym hervorgerufenen Umsetzung des Wasserstoffperoxids ein Messen der Trübung des behandelten Sterilisierungsmediums, um ein Reaktionsende zu bestimmen. In dem Verfahrensschritt kann beispielsweise ein Reaktionsende dann festgestellt werden, wenn ein gemessener Wert für die Trübung des behandelten Sterilisierungsmediums einen vorbestimmten Schwellenwert unterschreitet. Zum Messen der Trübung kann beispielsweise eine Extinktion und/oder Streuung einer das behandelte Sterilisierungsmedium durchsetzenden Strahlung bestimmt werden. Hierzu kann beispielsweise eine Strahlung im Infrarot-Wellenlängenbereich, d.h. Infrarot-Strahlung, verwendet werden.

Weiterhin wird eine Messvorrichtung zur Verwendung in einer Getränkeabfüllanlage vorgeschlagen, die zum Bestimmen einer Peressigsäure-Konzentration in einem durch die Getränkeabfüllanlage strömenden, peressigsäure- und wasserstoffperoxidhaltigen Sterilisierungsmedienstrom eingerichtet ist. Entsprechend umfasst die vorgeschlagene Messvorrichtung eine Zufuhreinheit zum Einspeisen eines Katalase-Enzyms in den durch die Getränkeabfüllanlage strömenden Sterilisierungsmedienstrom und eine stromabwärts der Zufuhreinheit angeordnete optische Messeinheit zum Bestimmen der Peressigsäure-Konzentration in dem Sterilisierungsmedienstrom. Vorliegend beziehen sich die Begriffe "stromabwärts" und "stromaufwärts" auf eine Strömungsrichtung des Sterilisierungsmedienstroms.

Die Messvorrichtung ist insbesondere dafür vorgesehen, das vorangehend beschriebene Verfahren zum Bestimmen einer Peressigsäure-Konzentration in einem peressigsäure- und wasserstoffperoxidhaltigen Sterilisierungsmedium auszuführen. Voranstehende, auf das Verfahren gerichtete technische Merkmale betreffen somit entsprechend die hier beschriebene Messvorrichtung zum Ausführen des Verfahrens.

Die Messvorrichtung ist dafür vorgesehen, einen durch eine Prozessleitung eines Reinigungssystems der Getränkeabfüllanlage strömenden Sterilisierungsmedienstrom zu analysieren. Die Prozessleitung kann dafür vorgesehen sein, einen Sterilisierungsmedienstrom durch die Getränkeabfüllanlage zu leiten, der einem Reinraum der Getränkeabfüllanlage zuzuführen und/oder aus diesem abzuführen ist. Das Reinigungssystem der Getränkeabfüllanlage kann insbesondere einen Kreislauf des Sterilisierungsmediums aufweisen, in dem das Sterilisierungsmedium bzw. der Sterilisierungsmedienstrom in der Getränkeabfüllanlage durch die Prozessleitung zirkuliert.

Die Zufuhreinheit ist zum Einspeisen des Katalase-Enzyms in die von dem Sterilisierungsmedienstrom durchströmte Prozessleitung eingerichtet. Entsprechend ist die optische Messeinheit dazu eingerichtet, den durch die Prozessleitung strömenden Sterilisierungsmedienstrom an einer Stelle der Prozessleitung zu analysieren, die bezogen auf die Durchflussrichtung des Sterilisierungsmedienstroms durch die Prozessleitung stromabwärts der Zufuhreinheit bzw. stromabwärts einer Position, an der das Katalase-Enzym dem Sterilisierungsmedienstrom zugeführt wird, angeordnet ist.

Die vorgeschlagene Messvorrichtung ermöglicht demnach, dass ein durch eine Prozessleitung der Getränkeabfüllanlage strömender Sterilisierungsmedienstrom kontinuierlich und nahezu ohne zeitliche Verzögerung analysiert werden kann. Auf diese Weise wird eine Messvorrichtung zur Inline-Messung einer Peressigsäure-Konzentration in einem peressigsäure- und wasserstoffperoxidhaltigen Sterilisierungsmedienstrom bereitgestellt.

Alternativ oder zusätzlich kann die Messvorrichtung dazu eingerichtet sein, einen durch eine Bypass-Leitung der Prozessleitung strömenden Sterilisierungsmedium-Bypass-Strom zu analysieren. In diesem Zusammenhang wird unter einer Bypass-Leitung eine zu der eigentlichen Prozessleitung parallel geschaltete Leitung verstanden, die eine geringe Menge des durch die Prozessleitung strömenden Sterilisierungsmedienstroms abzweigt, durch die Bypass-Leitung führt und anschließend der Prozessleitung wieder zuführt. Diese Ausgestaltung der Messvorrichtung ermöglicht eine Online-Messung des Sterilisierungsmedienstroms. Indem der durch die Bypass-Leitung strömende Sterilisierungsmedium-Bypass-Strom einen geringeren Massenstrom als der durch die Prozessleitung strömende Sterilisierungsmedienstrom aufweist, kann entsprechend die Menge von Katalase-Enzym reduziert werden, die dem Sterilisierungsmedium zum Bestimmen der Peressigsäure-Konzentration zuzuführen ist. Auf diese Weise lässt sich die Effizienz der Messvorrichtung und des Messverfahrens erhöhen. Die an dem durch die Bypass-Leitung strömenden Sterilisierungsmedium-Bypass-Strom durchgeführte Analyse mittels der Messvorrichtung ist dabei repräsentativ für den durch die Prozessleitung strömenden Sterilisierungsmedienstrom.

Zum Bestimmen der Peressigsäure-Konzentration in dem Sterilisierungsmedienstrom kann die optische Messeinheit dazu eingerichtet sein, die Peressigsäure-Konzentration in Abhängigkeit einer Extinktion von den Sterilisierungsmedienstrom durchleuchtender Strahlung, insbesondere UV-Strahlung, zu bestimmen. Entsprechend kann die optische Messeinheit eine Strahlungsquelle zum Emittieren von den Sterilisierungsmedienstrom durchleuchtender Strahlung, insbesondere UV-Strahlung, und einen optischen Detektor, insbesondere Lichtsensor, zum Empfangen und Messen der von der Strahlungsquelle emittierten, den Sterilisierungsmedienstrom durchleuchtenden Strahlung umfassen. Die Strahlungsquelle und der Lichtsensor können an gegenüberliegenden Seiten der Prozessleitung angeordnet sein, wobei die Prozessleitung im Bereich der optischen Messeinheit aus einem für die von der Strahlungsquelle emittierte Strahlung durchlässigen Material hergestellt sein kann. Die optische Messeinheit weist den Effekt auf, dass ein berührungsloses Messen der Peressigsäure-Konzentration in dem Sterilisierungsmedienstrom ermöglicht wird.

Um eine genaue Bestimmung der Peressigsäure-Konzentration in dem mit dem Katalase-Enzym versetzten Sterilisierungsmedium zu ermöglichen, kann die Messvorrichtung mit einer zwischen der Zufuhreinheit und der optischen Messeinheit angeordneten Verweilstrecke versehen sein. Die Verweilstrecke ist vorzugsweise von dem Sterilisierungsmedienstrom von der Zufuhreinheit in Richtung der optischen Messeinheit durchströmbar. Die Verweilstrecke weist den Effekt auf, dass nach dem Zuführen des Katalase-Enzyms das behandelte Sterilisierungsmedium zunächst durch die Verweilstrecke strömt und somit eine bestimmte Verweildauer vergeht, bevor eine Messung der Peressigsäure-Konzentration in dem behandelten Sterilisierungsmedium mittels der optischen Messeinheit erfolgt. Auf diese Weise kann sichergestellt werden, dass ein vollständiger Abbau von in dem Sterilisierungsmedium enthaltenem Wasserstoffperoxid erfolgt, bevor dieses von der optischen Messeinheit analysiert wird. Die Länge und Durchflussquerschnittsfläche der Verweilstrecke sind dabei derart gewählt, dass durch die Verweilstrecke strömendes Sterilisierungsmedium eine vorbestimmte Verweilzeit benötigt, um von der Zufuhreinheit in Richtung der optischen Messeinheit zu strömen. Die Verweilzeit wird derart gewählt, dass nach deren Ablauf ein vollständiger Abbau von Wasserstoffperoxid in dem Sterilisierungsmedienstrom mittels des Katalase-Enzyms sichergestellt werden kann. Die Durchflussquerschnittsfläche der Verweilstrecke entspricht dabei vorzugsweise einer Durchflussquerschnittsfläche einer stromaufwärts bzw. stromabwärts davon angeordneten Leitung. Auf diese Weise können Strömungsverluste in der Messvorrichtung vermieden werden. Alternativ kann die Durchflussquerschnittsfläche der Verweilstrecke größer sein als die der daran angrenzenden, stromaufwärts bzw. stromabwärts davon angeordneten Leitung, um die Verweildauer zu erhöhen. Die Verweilstrecke kann beispielsweise eine Länge von 2 m bis 25m, beispielsweise eine Länge von im Wesentlichen 5 m aufweisen.

Die Messvorrichtung kann ferner eine Durchfluss-Messeinheit umfassen, die zum Bestimmen eines Durchflusses, insbesondere eines Massen- und/oder Volumenstroms, des durch die Messvorrichtung bzw. die Prozessleitung der Getränkeabfüllanlage strömenden Sterilisierungsmedienstroms eingerichtet ist. Die Durchfluss-Messeinheit kann mit einer Steuereinheit der Messvorrichtung oder der Getränkeabfüllanlage verbunden oder verbindbar sein. Die Steuereinheit kann dazu eingerichtet sein, in Erwiderung auf den von der Durchfluss-Messeinheit bestimmten Wert für den Durchfluss des Sterilisierungsmedienstroms und in Abhängigkeit der Länge und der Durchflussquerschnittsfläche der Verweilstrecke eine Verweildauer des Sterilisierungsmediums zwischen der Zufuhreinheit und der optischen Messeinheit zu bestimmen.

Erfindungsgemäß umfasst die Messvorrichtung eine stromabwärts der Zufuhreinheit angeordnete Trübungs-Messeinheit. Die Trübungs-Messeinheit ist zum Bestimmen eines Reaktionsendes der durch das Katalase-Enzym hervorgerufenen Umsetzung von Wasserstoffperoxid in dem Sterilisierungsmedienstrom eingerichtet. Genauer ist die Trübungs-Messeinheit zum Messen einer Trübung des Sterilisierungsmedienstroms eingerichtet. Hierzu kann die Trübungs-Messeinheit eine Lichtquelle zum Emittieren von den Sterilisierungsmedienstrom durchleuchtender Infrarot-Strahlung und einen optischen Detektor, insbesondere einen Lichtsensor, zum Messen der durch den Sterilisierungsmedienstrom tretenden Infrarot-Strahlung umfassen. Ferner kann die Trübungs-Messeinheit dazu eingerichtet sein, ein Reaktionsende der Umsetzung von Wasserstoffperoxid in dem Sterilisierungsmedienstrom zu bestimmen, wenn ein gemessener Wert für die Trübung einen vorgegebenen Schwellenwert unterschreitet.

Die optische Messeinheit kann dazu eingerichtet sein, die Peressigsäure-Konzentration in dem Sterilisierungsmedienstrom zu bestimmen, wenn ein Reaktionsende der durch das Katalase-Enzym hervorgerufenen Umsetzung von Wasserstoffperoxid in dem Sterilisierungsmedienstrom durch die weitere optische Messeinheit angezeigt oder bestimmt wird. Auf diese Weise kann verhindert werden, dass eine Messung mittels der optischen Messeinheit in einem Zustand erfolgt, in dem ein vollständiger Abbau von Wasserstoffperoxid in dem Sterilisierungsmedienstrom noch nicht erreicht ist.

Ferner können/kann die optische Messeinheit und/oder die Zufuhreinheit mit der Steuereinheit der Messvorrichtung verbunden sein und/oder mit der Steuereinheit der Getränkeabfüllanlage verbindbar sein. Dabei kann die Steuereinheit dazu eingerichtet sein, einen Zufluss von Peressigsäure und/oder des Katalase-Enzyms in den Sterilisierungsmedienstrom in Abhängigkeit der durch die optische Messeinheit bestimmten Peressigsäure-Konzentration zu steuern. Der Zufluss von Peressigsäure kann hierbei mittels einer weiteren Zufuhreinheit verwirklicht sein und dazu dienen, den Sterilisierungsmedienstrom aufzubereiten, bevor dieser zur Durchführung einer Sterilisation einer Getränkeabfüllanlage zugeführt wird.

In einer Weiterentwicklung kann die Messvorrichtung ferner eine stromaufwärts der Zufuhreinheit angeordnete weitere optische Messeinheit zum Bestimmen einer Konzentration von Peressigsäure und Wasserstoffperoxid in dem Sterilisierungsmedienstrom umfassen. Die weitere optische Messeinheit kann entsprechend der voranstehend offenbarten optischen Messeinheit ausgestaltet sein. Die weitere optische Messeinheit kann mit der Steuereinheit verbunden sein. Entsprechend kann die Steuereinheit dazu eingerichtet sein, in Abhängigkeit der durch die optische Messeinheit bestimmten Peressigsäure-Konzentration in dem Sterilisierungsmedienstrom stromabwärts der Zufuhreinheit und der durch die weitere optische Messeinheit bestimmten Konzentration von Peressigsäure und Wasserstoffperoxid in dem Sterilisierungsmedienstrom stromaufwärts der Zufuhreinheit eine Wasserstoffperoxid-Konzentration in dem Sterilisierungsmedienstrom stromaufwärts der Zufuhreinheit zu bestimmen. Genauer kann dies erfolgen, indem die durch die optische Messeinheit bestimmte Peressigsäure-Konzentration von der durch die weitere optische Messeinheit bestimmten Konzentration von Peressigsäure und Wasserstoffperoxid subtrahiert wird. Auf diese Weise ermöglicht die vorgeschlagene Messvorrichtung weiterhin ein Bestimmen der Wasserstoffperoxid-Konzentration in einem peressigsäure- und wasserstoffperoxidhaltigen Sterilisierungsmedienstrom.

Ferner wird eine Getränkeabfüllanlage vorgeschlagen, die ein Reinigungssystem zum Bereitstellen eines durch die Getränkeabfüllanlage strömenden Sterilisierungsmedienstroms und die vorangehend beschriebene Messvorrichtung zum Bestimmen einer Peressigsäure-Konzentration in dem durch die Getränkeabfüllanlage fließenden Sterilisierungsmedienstrom umfasst.

Das Reinigungssystem ist vorzugsweise dazu eingerichtet, den Sterilisierungsmedienstrom in einem Kreislauf innerhalb der Getränkeabfüllanlage zu zirkulieren, bei dem Sterilisierungsmedium in einem Reinraum der Getränkeabfüllanlage aufgesammelt, aufbereitet und daraufhin dem Reinraum erneut zugeführt wird. Auf diese Weise kann das Sterilisierungsmedium wiederverwertet und so die Effizienz der Getränkeabfüllanlage erhöht werden.

In einer Weiterentwicklung kann der Messvorrichtung ein von einer Prozessleitung des Reinigungssystems abgezweigter, durch eine Bypass-Leitung strömender Sterilisierungsmedien-Bypass-Strom zuführbar sein. Entsprechend kann die Messvorrichtung dazu eingerichtet sein, den durch die Bypass-Leitung strömenden Sterilisierungsmedien-Bypass-Strom mittels der Zufuhreinheit zu behandeln und mittels der optischen Messeinheit zu analysieren, um die Peressigsäure-Konzentration in dem durch das Reinigungssystem zirkulierenden Sterilisierungsmedienstroms zu bestimmen.

### Kurze Beschreibung der Figuren

Bevorzugte weitere Ausführungsformen der Erfindung werden durch die nachfolgende Beschreibung der Figuren näher erläutert. Dabei zeigen schematisch:
Figur 1 eine Getränkeabfüllanlage mit einer Messvorrichtung einer ersten Ausführungsform; und
Figur 2 die Getränkeabfüllanlage mit einer Messvorrichtung einer zweiten Ausführungsform.

### Detaillierte Beschreibung bevorzugter Ausführungsbeispiele

Im Folgenden werden bevorzugte Ausführungsbeispiele anhand der Figuren beschrieben. Dabei werden gleiche, ähnliche oder gleichwirkende Elemente in den unterschiedlichen Figuren mit identischen Bezugszeichen versehen, und auf eine wiederholte Beschreibung dieser Elemente wird teilweise verzichtet, um Redundanzen zu vermeiden.

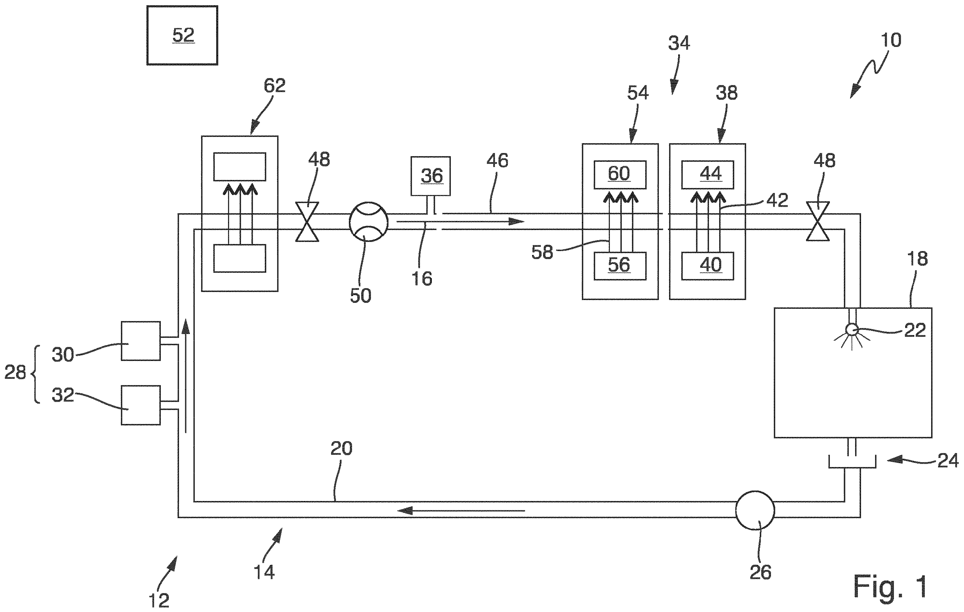

In Figur 1 ist eine Getränkeabfüllanlage 10 gezeigt, die ein Reinigungssystem 12 zum Bereitstellen und Aufbereiten eines durch die Getränkeabfüllanlage 10 in einem Kreislauf 14 zirkulierenden Sterilisierungsmedienstroms 16 umfasst. Die Getränkeabfüllanlage 10 umfasst einen Reinraum 18, in dem eine Produktionslinie der Getränkeabfüllanlage 10 angeordnet ist und Prozessgut verarbeitet wird. Zum Sicherstellen von sterilen Bedingungen in der Getränkeabfüllanlage 10 wird dem Reinraum 18 ein Sterilisierungsmedium über eine Prozessleitung 20 des Reinigungssystems 12 während des Betriebs zugeführt und darin über Verteildüsen 22 versprüht. Das den Sterilisierungsmedienstrom 16 bildende Sterilisierungsmedium weist Peressigsäure und Wasserstoffperoxid auf.

Genauer ist das Reinigungssystem 12 dazu eingerichtet, Sterilisierungsmedium in dem Reinraum 18 zu versprühen, das darin versprühte Sterilisierungsmedium in einer Auffangeinheit 24 einzusammeln, aufzubereiten und daraufhin erneut dem Reinraum 18 zuzuführen. Auf diese Weise erfolgt eine Wiederverwertung des Sterilisierungsmediums, wodurch die Effizienz der Getränkeabfüllanlage 10 erhöht wird. In der Auffangeinheit 24 eingesammeltes Sterilisierungsmedium wird über eine Förderpumpe 26 der Prozessleitung 20 erneut zugeführt und in Richtung einer Aufbereitungseinheit 28 geleitet.

Die Aufbereitungseinheit 28 umfasst eine Peressigsäure-Versorgungseinheit 30 und eine Wasser-Versorgungseinheit 32, die zum Einspeisen von Peressigsäure und Wasser in den durch die Prozessleitung 20 fließenden Sterilisierungsmedienstrom 16 eingerichtet sind. Insbesondere erfolgt in der hier gezeigten Getränkeabfüllanlage 10 ein Aufbereiten des Sterilisierungsmedienstroms 16 durch Hinzufügen von Peressigsäure und/oder Wasser, um eine Peressigsäure-Konzentration in dem Sterilisierungsmedienstrom 16 einzustellen und damit die Reinigungswirkung des Sterilisierungsmediums zu beeinflussen.

Um ein zielgerichtetes Einstellen der Peressigsäure-Konzentration in dem Sterilisierungsmedienstrom 16 zu ermöglichen, umfasst die Getränkeabfüllanlage 10 ferner eine Messvorrichtung 34 zum Bestimmen der Peressigsäure-Konzentration in dem durch die Getränkeabfüllanlage 10 strömenden, peressigsäure- und wasserstoffperoxidhaltigen Sterilisierungsmedienstrom 16.

Die Messvorrichtung 34 umfasst eine Zufuhreinheit 36 zum Einspeisen eines Katalase-Enzyms in den durch die Prozessleitung 20 strömenden Sterilisierungsmedienstrom 16. Mit anderen Worten, mittels der Zufuhreinheit 36 erfolgt ein Behandeln des Sterilisierungsmedienstroms 16 durch Zuführen des Katalase-Enzyms, das in dem Sterilisierungsmedienstrom 16 enthaltenes Wasserstoffperoxid zu Sauerstoff und Wasser umsetzt.

Der derart behandelte Sterilisierungsmedienstrom 16 fließt dann von der Zufuhreinheit 36 in Richtung einer stromabwärts der Zufuhreinheit 36 angeordneten optischen Messeinheit 38, die zum Bestimmen der Peressigsäure-Konzentration in dem behandelten Sterilisierungsmedienstrom 16 mittels eines optischen Messverfahrens eingerichtet ist. Genauer umfasst die optische Messeinheit 38 eine Strahlungsquelle 40 zum Emittieren von den Sterilisierungsmedienstrom 16 durchleuchtender UV-Strahlung 42 mit einem Wellenlängenbereich zwischen 200 nm und 300 nm. Hierzu umfasst die Prozessleitung 20 im Bereich der optischen Messeinheit 38 einen für die UV-Strahlung 42 durchlässigen Abschnitt. Auf diese Weise durchquert die von der Strahlungsquelle 40 emittierte UV-Strahlung 42 den durch die Prozessleitung 20 im Bereich der Messeinheit 38 strömenden Sterilisierungsmedienstrom 16 und trifft daraufhin auf einen UV-Lichtsensor 44 der optischen Messeinheit 38. Der UV-Lichtsensor 44 ist zum Messen der den Sterilisierungsmedienstrom 16 durchsetzenden UV-Strahlung 42 eingerichtet. Im Speziellen handelt es sich bei dem UV-Lichtsensor 44 um ein elektronisches Bauelement, dass die einfallende UV-Strahlung 42 unter Benutzung des photoelektrischen Effekts in ein elektrisches Signal umwandelt oder einen von der einfallenden UV-Strahlung 42 abhängigen elektrischen Widerstand anzeigt. Entsprechend misst der UV-Lichtsensor 44 die Intensität der einfallenden UV-Strahlung 42 und kann so eine Extinktion, d.h. Abschwächung der auf den UV-Lichtsensor 44 auftreffenden UV-Strahlung 42 bestimmen. Die Messvorrichtung 34 ist dabei dazu eingerichtet, in Abhängigkeit der durch die optische Messeinheit 38 ermittelten Extinktion die Peressigsäure-Konzentration in dem Sterilisierungsmedienstrom 16 zu bestimmen.

Um genaue Messergebnisse zu gewährleisten, ist in der Messvorrichtung 34 eine definierte, durch gestrichelte Linien in Figur 1 angezeigte Verweilstrecke 46 zwischen der Zufuhreinheit 36 und der optischen Messeinheit 38 angeordnet, durch die der Sterilisierungsmedienstrom 16 stromabwärts der Zufuhreinheit 36 bis zu der optischen Messeinheit 38 fließt. Die Verweilstrecke 46 kann beispielsweise eine Länge von 5 m aufweisen und hat eine Strömungsquerschnittsfläche, die im Wesentlichen denen der daran angrenzenden Abschnitte der Prozessleitung 20 entspricht. Die Verweilstrecke 46 weist den Effekt auf, dass nach Zuführen des Katalase-Enzyms das behandelte Sterilisierungsmedium zunächst durch die Verweilstrecke 46 strömt und somit eine vorgegebene Verweildauer vergeht, bevor dieses die optische Messeinheit 38 erreicht und die Peressigsäure-Konzentration gemessen wird. Die Messvorrichtung 34 ist dabei derart ausgebildet, dass die Verweildauer von Sterilisierungsmedium in der Verweilstrecke 46 ausreichend groß ist, dass eine vollständige Umsetzung des darin enthaltenen Wasserstoffperoxids mittels des Katalase-Enzyms sichergestellt werden kann. So kann verhindert werden, dass der die optische Messeinheit 38 durchströmende Sterilisierungsmedienstrom 16 Wasserstoffperoxid aufweist, das das optische Messverfahren beeinflussen könnte.

Zum Einstellen der Strömungsgeschwindigkeit des Sterilisierungsmedienstroms 16 durch die Messvorrichtung 34 und somit zum Einstellen der Verweildauer des Sterilisierungsmediums in der Verweilstrecke 46 umfasst die Messvorrichtung 34 an deren Ein- oder Auslass ein Durchfluss-Regelventil 48 in der Prozessleitung 20. Ferner umfasst die Messvorrichtung 34 eine Durchfluss-Messeinheit 50 zum Bestimmen eines Durchflusses, insbesondere eines Massen- und/oder Volumenstroms, des durch die Messvorrichtung 34 strömenden Sterilisierungsmedienstroms 16. Die Durchfluss-Messeinheit 50 ist mit einer Steuereinheit 52 verbunden, die in Erwiderung auf den durch die Durchfluss-Messeinheit 50 ermittelten Durchfluss eine Verweildauer des behandelten Sterilisierungsmediums in der Verweilstrecke 46 bestimmt. Diese Konfiguration der Messvorrichtung 34 ermöglicht ein genaues Einstellen der Verweildauer des Sterilisierungsmediums in der Verweilstrecke 46 im Betrieb des Reinigungssystems 12.

Die Messvorrichtung 34 umfasst ferner eine in Strömungsrichtung unmittelbar vor der optischen Messeinheit 38 angeordnete Trübungs-Messeinheit 54 zu Messen einer Trübung des Sterilisierungsmedienstroms 16. Die Trübungs-Messeinheit 54 umfasst eine Infrarot-Lichtquelle 56 zum Emittieren von den Sterilisierungsmedienstrom 16 durchleuchtender Infrarot-Strahlung 58 und einen Infrarot-Lichtsensor 60, auf den die Infrarot-Strahlung 58 nach Durchqueren des Sterilisierungsmedienstroms 16 trifft. Der Lichtsensor 60 misst dabei die Extinktion der den Sterilisierungsmedienstrom 16 durchsetzenden Infrarot-Strahlung 58, um die Trübung zu bestimmen. Genauer ist die Trübungs-Messeinheit 54 dazu eingerichtet, der Steuereinheit 52 ein Reaktionsende der durch das Katalase-Enzym hervorgerufenen Umsetzung von Wasserstoffperoxid in dem Sterilisierungsmedienstrom 16 anzuzeigen, wenn ein gemessener Wert für die Trübung einen vorgegebenen Schwellenwert unterschreitet. Mit anderen Worten, der Schwellenwert ist derart gewählt, dass bei dessen Unterschreitung von einem im Wesentlichen klaren, d.h. eine vernachlässigbare Trübung aufweisenden Sterilisierungsmedium ausgegangen werden kann, was einen Indikator für das Reaktionsende der Umsetzung von Wasserstoffperoxid darstellt. Um fehlerhaften Messungen der optischen Messeinheit 38 vorzubeugen, kann die Messvorrichtung 34 dazu eingerichtet sein, die Peressigsäure-Konzentration mittels der optischen Messeinheit 38 entsprechend nur dann zu bestimmen, wenn ein Reaktionsende in dem Sterilisierungsmedienstrom 16 durch die Trübungs-Messeinheit 54 bestimmt wird.

Die Trübungs-Messeinheit 54 und die optische Messeinheit können alternativ in einer einzigen Einheit aufgebaut sein.

Die optische Messeinheit 38 ist mit der Steuereinheit 52 verbunden und dazu eingerichtet, dieser die durch das optische Messverfahren bestimmten Werte für die Peressigsäure-Konzentration in dem Sterilisierungsmedienstrom 16 zu übermitteln. Die Steuereinheit 52 ist dazu eingerichtet, in Abhängigkeit der durch die optische Messeinheit 38 bestimmten Peressigsäure-Konzentration die Zufuhreinheit 36, die Peressigsäure-Versorgungseinheit 30 und die Wasser-Versorgungseinheit 32 zu steuern, um einen Zufluss von Peressigsäure und/oder Wasser und/oder des Katalase-Enzyms in den Sterilisierungsmedienstrom 16 einzustellen.

Ferner umfasst die Messvorrichtung 34 eine stromaufwärts der Zufuhreinheit 36 angeordnete weitere optische Messeinheit 62 zum Bestimmen einer Konzentration von Peressigsäure und Wasserstoffperoxid in dem Sterilisierungsmedienstrom 16 stromaufwärts der Zufuhreinheit 36. Die weitere optische Messeinheit 62 ist entsprechend der optischen Messeinheit 38 ausgebildet und mit der Steuereinheit 52 zum Übermitteln von Messergebnissen verbunden. Die Steuereinheit 52 ist ferner dazu eingerichtet, eine Wasserstoffperoxid-Konzentration in dem Sterilisierungsmedienstrom 16 stromaufwärts der Zufuhreinheit 36 zu bestimmen. Dies erfolgt durch Subtraktion der durch die optische Messeinheit 38 bestimmten Peressigsäure-Konzentration in dem Sterilisierungsmedienstrom 16 stromabwärts der Zufuhreinheit 36 von der durch die weitere optische Messeinheit 62 bestimmten Konzentration von Peressigsäure und Wasserstoffperoxid in dem Sterilisierungsmedienstrom 16 stromaufwärts der Zufuhreinheit 36.

Im Ergebnis ermöglicht die hier gezeigte Messvorrichtung 34, dass der durch die Prozessleitung 20 strömende Sterilisierungsmedienstrom 16 kontinuierlich und nahezu ohne zeitliche Verzögerung analysiert werden kann. Auf diese Weise werden ein Verfahren und eine Vorrichtung zur Inline-Messung der Peressigsäure- und/oder Wasserstoffperoxid-Konzentration in einem peressigsäure- und wasserstoffperoxidhaltigen Sterilisierungsmedienstrom bereitgestellt.

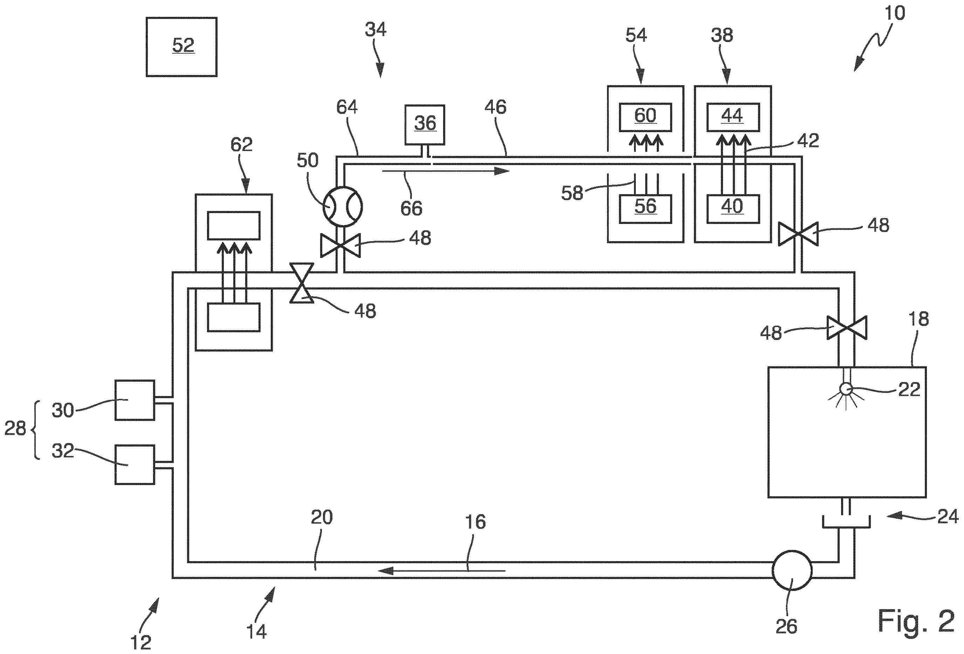

Figur 2 zeigt eine weitere Getränkeabfüllanlage 10, bei der der Messvorrichtung 34 im Vergleich zu der in Figur 1 gezeigten Messvorrichtung ein von der Prozessleitung 20 abgezweigter, durch eine Bypass-Leitung 64 strömender Sterilisierungsmedium-Bypass-Strom 66 zugeführt wird. Entsprechend ist die Messvorrichtung 34 dazu eingerichtet, den durch die Bypass-Leitung 64 strömenden Sterilisierungsmedien-Bypass-Strom 66 mittels der Zufuhreinheit 36 zu behandeln und mittels der optischen Messeinheit 38 zu analysieren, um die Peressigsäure-Konzentration in dem durch das Reinigungssystem 12 zirkulierenden Sterilisierungsmedienstrom 16 zu bestimmen. Indem ein von dem durch die Prozessleitung 20 strömenden Sterilisierungsmedienstrom 16 abgezweigter Sterilisierungsmedium-Bypass-Strom 66 von der Messvorrichtung 34 analysiert wird, ermöglicht diese Konfiguration eine Online-Messung der Peressigsäure-Konzentration in dem Sterilisierungsmedium.

### Bezugszeichenliste

- 10: Getränkeabfüllanlage
- 12: Reinigungssystem
- 14: Kreislauf für ein Sterilisierungsmedium
- 16: Sterilisierungsmedienstrom
- 18: Reinraum
- 20: Prozessleitung
- 22: Verteildüse
- 24: Auffangeinheit
- 26: Förderpumpe
- 28: Aufbereitungseinheit
- 30: Peressigsäure-Versorgungseinheit
- 32: Wasser-Versorgungseinheit
- 34: Messvorrichtung
- 36: Zufuhreinheit
- 38: Optische Messeinheit
- 40: Strahlungsquelle
- 42: UV-Strahlung
- 44: UV-Lichtsensor
- 46: Verweilstrecke
- 48: Regelventil
- 50: Durchfluss-Messeinheit
- 52: Steuereinheit
- 54: Trübungs-Messeinheit
- 56: Infrarot-Lichtquelle
- 58: Infrarot-Strahlung
- 60: Infrarot-Lichtsensor
- 62: Weitere optische Messeinheit
- 64: Bypass-Leitung
- 66: Sterilisierungsmedium-Bypass-Strom

## Patentansprüche

1. Verfahren zum Bestimmen einer Peressigsäure-Konzentration in einem peressigsäure- und wasserstoffperoxidhaltigen Sterilisierungsmedium in einer Getränkeabfüllanlage (10), umfassend die Schritte:
- Behandeln des Sterilisierungsmediums durch Zuführen eines Katalase-Enzyms zur Umsetzung von in dem Sterilisierungsmedium enthaltenem Wasserstoffperoxid zu Sauerstoff und Wasser;
- Ermitteln des Reaktionsendes der durch das Katalase-Enzym hervorgerufenen Umsetzung von Wasserstoffperoxid; und
- Bestimmen der Peressigsäure-Konzentration in dem behandelten Sterilisierungsmedium mittels eines optischen Messverfahrens nach dem ermittelten Reaktionsende der durch das Katalase-Enzym hervorgerufenen Umsetzung, wobei
in dem Schritt des Ermittelns des Reaktionsendes der durch das Katalase-Enzym hervorgerufenen Umsetzung eine Trübung des behandelten Sterilisierungsmediums gemessen wird.

2. Verfahren nach Anspruch 1, wobei in dem optischen Messverfahren eine Extinktion einer das behandelte Sterilisierungsmedium durchsetzenden Strahlung (42), insbesondere UV-Strahlung, ermittelt wird und die Peressigsäure-Konzentration in Abhängigkeit der ermittelten Extinktion bestimmt wird.

3. Verfahren nach Anspruch 1 oder 2, wobei in dem Schritt des Ermittelns des Reaktionsendes ein Reaktionsende festgestellt wird, wenn ein gemessener Wert für die Trübung einen vorbestimmten Schwellenwert unterschreitet, wobei insbesondere zum Messen der Trübung des behandelten Sterilisierungsmediums eine Extinktion und/oder Streuung einer das behandelte Sterilisierungsmedium durchsetzenden weiteren Strahlung (58), insbesondere Infrarot-Strahlung, bestimmt werden/wird.

4. Messvorrichtung (34) zur Verwendung in einer Getränkeabfüllanlage (10) und zum Bestimmen einer Peressigsäure-Konzentration in einem durch die Getränkeabfüllanlage (10) strömenden, peressigsäure- und wasserstoffperoxidhaltigen Sterilisierungsmedienstrom (16), umfassend:
- eine Zufuhreinheit (36) zum Einspeisen eines Katalase-Enzyms in den durch die Getränkeabfüllanlage (10) strömenden Sterilisierungsmedienstrom (16; 66) und
- eine stromabwärts der Zufuhreinheit (36) angeordnete optische Messeinheit (38) zum Bestimmen der Peressigsäure-Konzentration in dem Sterilisierungsmedienstrom (16; 66), **dadurch gekennzeichnet, dass** die Messvorrichtung
- eine stromabwärts der Zufuhreinheit (36) angeordnete Trübungs-Messeinheit (54), die zum Messen einer Trübung des Sterilisierungsmedienstroms (16; 66) eingerichtet ist, zum Bestimmen eines Reaktionsendes einer durch das Katalase-Enzym hervorgerufenen Umsetzung von Wasserstoffperoxid in dem Sterilisierungsmedienstrom zu Sauerstoff und Wasser (16; 66) umfasst.

5. Messvorrichtung nach Anspruch 4, **dadurch gekennzeichnet, dass** die optische Messeinheit (38) eine Strahlungsquelle (40) zum Emittieren von den Sterilisierungsmedienstrom (16; 66) durchsetzender Strahlung (42), insbesondere UV-Strahlung, und einen Lichtsensor (44) zum Messen der den Sterilisierungsmedienstrom (16; 66) durchsetzenden Strahlung (42) umfasst.

6. Messvorrichtung nach Anspruch 4 oder 5, wobei die Messvorrichtung eine zwischen der Zufuhreinheit (36) und der optischen Messeinheit (38) angeordnete Verweilstrecke (46) umfasst, die von dem Sterilisierungsmedienstrom (16) von der Zufuhreinheit (36) in Richtung der optischen Messeinheit (38) durchströmbar ist, wobei insbesondere die Verweilstrecke (46) eine Länge von mindestens 2 m bis 25m, insbesondere eine Länge von im Wesentlichen 5 m, aufweist.

7. Messvorrichtung nach einem der Ansprüche 4 bis 6, **dadurch gekennzeichnet, dass** sie eine Durchfluss-Messeinheit (50) zum Bestimmen eines Durchflusses, insbesondere eines Massen- und/oder Volumenstroms, des durch die Messvorrichtung (34) strömenden Sterilisierungsmedienstroms (16; 66) umfasst.

8. Messvorrichtung nach einem der Ansprüche 4 bis 7, **dadurch gekennzeichnet, dass** die Trübungs-Messeinheit (54) eine weitere Lichtquelle (56) zum Emittieren von den Sterilisierungsmedienstrom durchleuchtender Infrarot-Strahlung (58) und einen weiteren Lichtsensor (56) zum Messen der durch den Sterilisierungsmedienstrom (16; 66) tretenden Infrarot-Strahlung (58) umfasst, und wobei insbesondere die Trübungs-Messeinheit (54) dazu eingerichtet ist, ein Reaktionsende zu bestimmen, wenn ein gemessener Wert für die Trübung einen vorgegebenen Schwellenwert unterschreitet.

9. Messvorrichtung nach Anspruch 8, **dadurch gekennzeichnet, dass** die optische Messeinheit (38) dazu eingerichtet ist, die Peressigsäure-Konzentration in dem Sterilisierungsmedienstrom (16; 66) zu bestimmen, wenn ein Reaktionsende der durch das Katalase-Enzym hervorgerufenen Umsetzung von Wasserstoffperoxid in dem Sterilisierungsmedienstrom (16; 66) durch die Trübungs-Messeinheit (54) bestimmt wird.

10. Messvorrichtung nach einem der Ansprüche 4 bis 9, wobei die Messvorrichtung eine Steuereinheit (52) umfasst, mit der die optische Messeinheit (38) und/oder die Zufuhreinheit (36) verbunden sind/ist, die einen Zufluss von Peressigsäure und/oder des Katalase-Enzyms in den Sterilisierungsmedienstrom (16; 66) in Abhängigkeit der durch die optische Messeinheit (38) bestimmten Peressigsäure-Konzentration steuert.

11. Messvorrichtung nach Anspruch 10, **dadurch gekennzeichnet, dass** sie eine stromaufwärts der Zufuhreinheit (36) angeordnete weitere optische Messeinheit (62) zum Bestimmen einer Konzentration von Peressigsäure und Wasserstoffperoxid in dem Sterilisierungsmedienstrom (16) umfasst, die mit der Steuereinheit (52) verbunden ist, wobei die Steuereinheit (52) dazu eingerichtet ist, in Abhängigkeit der durch die optische Messeinheit (38) bestimmten Peressigsäure-Konzentration in dem Sterilisierungsmedienstrom (16; 66) stromabwärts der Zufuhreinheit (36) und der durch die weitere optische Messeinheit (62) bestimmten Konzentration von Peressigsäure und Wasserstoffperoxid in dem Sterilisierungsmedienstrom (16) stromaufwärts der Zufuhreinheit (36) eine Wasserstoffperoxid-Konzentration in dem Sterilisierungsmedienstrom (16) stromaufwärts der Zufuhreinheit (36) zu bestimmen.

12. Getränkeabfüllanlage (10), umfassend ein Reinigungssystem (12) zum Bereitstellen eines durch die Getränkeabfüllanlage fließenden Sterilisierungsmedienstroms (16) und eine Messvorrichtung (34) nach einem der Ansprüche 4 bis 11 zum Bestimmen einer Peressigsäure-Konzentration in dem durch die Getränkeabfüllanlage fließenden Sterilisierungsmedienstrom (16).

13. Getränkeabfüllanlage nach Anspruch 12, **dadurch gekennzeichnet, dass** der Messvorrichtung (34) ein durch eine Bypass-Leitung (64) strömender Sterilisierungsmedium-Bypass-Strom (66) zuführbar ist und die Messvorrichtung (34) dazu eingerichtet ist, den durch die Bypass-Leitung (64) strömenden Sterilisierungsmedium-Bypass-Strom (66) mittels der Zufuhreinheit (36) zu behandeln und mittels der optischen Messeinheit (38) zu analysieren, um die Peressigsäure-Konzentration in dem Sterilisierungsmedienstrom (16) zu bestimmen.

## Claims

1. Method for determining a peracetic acid concentration in a sterilising medium containing peracetic acid and hydrogen peroxide in a beverage filling plant (10), comprising the steps of:
- treating the sterilising medium by supplying a catalase enzyme for converting hydrogen peroxide contained in the sterilising medium into oxygen and water;
- ascertaining the end of the reaction of the conversion of hydrogen peroxide caused by the catalase enzyme; and
- determining the peracetic acid concentration in the treated sterilising medium by means of an optical measuring method after the ascertained end of the reaction of the conversion caused by the catalase enzyme, wherein
in the step of ascertaining the end of the reaction of the conversion caused by the catalase enzyme, a turbidity of the treated sterilising medium is measured.

2. Method according to claim 1, wherein, in the optical measuring method, an extinction of radiation (42) penetrating the treated sterilising medium, in particular UV radiation, is ascertained and the peracetic acid concentration is determined as a function of the ascertained extinction.

3. Method according to claim 1 or 2, wherein, in the step of ascertaining the end of the reaction, an end of the reaction is established when a measured value for the turbidity falls below a predetermined threshold value, wherein in particular for measuring the turbidity of the treated sterilising medium, an extinction and/or scattering of further radiation (58) penetrating the treated sterilising medium, in particular infrared radiation, is/are determined.

4. Measuring device (34) for use in a beverage filling plant (10) and for determining a peracetic acid concentration in a sterilising medium flow (16) which contains peracetic acid and hydrogen peroxide and flows through the beverage filling plant (10), comprising:
- a supply unit (36) for feeding a catalase enzyme into the sterilising medium flow (16; 66) flowing through the beverage filling plant (10), and
- an optical measuring unit (38) arranged downstream of the supply unit (36) for determining the peracetic acid concentration in the sterilising medium flow (16; 66),
**characterised in that** the measuring device comprises:
- a turbidity measuring unit (54) which is arranged downstream of the supply unit (36) and configured to measure a turbidity of the sterilising medium flow (16; 66), for determining an end of the reaction of a conversion of hydrogen peroxide in the sterilising medium flow, which conversion is caused by the catalase enzyme, into oxygen and water (16; 66).

5. Measuring device according to claim 4, **characterised in that** the optical measuring unit (38) comprises a radiation source (40) for emitting radiation (42) penetrating the sterilising medium flow (16; 66), in particular UV radiation, and a light sensor (44) for measuring the radiation (42) penetrating the sterilising medium flow (16; 66).

6. Measuring device according to claim 4 or 5, wherein the measuring device comprises a dwell section (46) which is arranged between the supply unit (36) and the optical measuring unit (38) and through which the sterilising medium flow (16) can flow from the supply unit (36) in the direction of the optical measuring unit (38), wherein in particular the dwell section (46) has a length of at least 2 m to 25 m, in particular a length of substantially 5 m.

7. Measuring device according to any of claims 4 to 6, **characterised in that** it comprises a flow rate measuring unit (50) for determining a flow rate, in particular of a mass and/or volume flow, of the sterilising medium flow (16; 66) flowing through the measuring device (34).

8. Measuring device according to any of claims 4 to 7, **characterised in that** the turbidity measuring unit (54) comprises a further light source (56) for emitting infrared radiation (58) shining through the sterilising medium flow and a further light sensor (56) for measuring the infrared radiation (58) passing through the sterilising medium flow (16; 66), and wherein in particular the turbidity measuring unit (54) is configured to determine an end of the reaction when a measured value for the turbidity falls below a predefined threshold value.

9. Measuring device according to claim 8, **characterised in that** the optical measuring unit (38) is configured to determine the peracetic acid concentration in the sterilising medium flow (16; 66) when an end of the reaction of the conversion of hydrogen peroxide in the sterilising medium flow (16; 66) caused by the catalase enzyme is determined by the turbidity measuring unit (54).

10. Measuring device according to any of claims 4 to 9, wherein the measuring device comprises a control unit (52) to which the optical measuring unit (38) and/or the supply unit (36) are/is connected and which controls an inflow of peracetic acid and/or of the catalase enzyme into the sterilising medium flow (16; 66) as a function of the peracetic acid concentration determined by the optical measuring unit (38).

11. Measuring device according to claim 10, **characterised in that** it comprises a further optical measuring unit (62) arranged upstream of the supply unit (36) for determining a concentration of peracetic acid and hydrogen peroxide in the sterilising medium flow (16), which unit is connected to the control unit (52), wherein the control unit (52) is configured to determine, as a function of the peracetic acid concentration in the sterilising medium flow (16; 66) determined by the optical measuring unit (38) downstream of the supply unit (36) and the concentration of peracetic acid and hydrogen peroxide in the sterilising medium flow (16) determined by the further optical measuring unit (62) upstream of the supply unit (36), a hydrogen peroxide concentration in the sterilising medium flow (16) upstream of the supply unit (36).

12. Beverage filling plant (10), comprising a cleaning system (12) for providing a sterilising medium flow (16) flowing through the beverage filling plant and a measuring device (34) according to any of claims 4 to 11 for determining a peracetic acid concentration in the sterilising medium flow (16) flowing through the beverage filling plant.

13. Beverage filling plant according to claim 12, **characterised in that** the measuring device (34) can be supplied with a sterilising medium bypass flow (66) flowing through a bypass line (64) and the measuring device (34) is configured to treat the sterilising medium bypass flow (66) flowing through the bypass line (64) by means of the supply unit (36) and to analyse said flow by means of the optical measuring unit (38) in order to determine the peracetic acid concentration in the sterilising medium flow (16).

## Revendications

1. Procédé de détermination de la concentration d'acide peracétique dans un milieu de stérilisation contenant de l'acide peracétique et du peroxyde d'hydrogène dans une usine de remplissage de boissons (10), comprenant les étapes consistant à :
- traiter le milieu de stérilisation par apport d'une enzyme catalase pour convertir le peroxyde d'hydrogène contenu dans le milieu de stérilisation en oxygène et en eau ;
- identifier la fin de la réaction de conversion du peroxyde d'hydrogène provoquée par enzyme catalase ; et
- déterminer la concentration d'acide peracétique dans le milieu de stérilisation traité au moyen d'un procédé de mesure optique après la fin de la réaction identifiée de la réaction provoquée par enzyme catalase, dans lequel
lors de l'étape de détermination de la fin de réaction de la conversion provoquée par enzyme catalase, une turbidité du milieu de stérilisation traité est mesurée.

2. Procédé selon la revendication 1, dans lequel
une extinction d'un rayonnement (42) traversant le milieu de stérilisation traité, en particulier un rayonnement UV, est identifiée dans le procédé de mesure optique, et la concentration d'acide peracétique est déterminée en fonction de l'extinction identifiée.

3. Procédé selon la revendication 1 ou 2, dans lequel
lors de l'étape de détermination de la fin de réaction, une fin de la réaction est établie lorsqu'une valeur mesurée de la turbidité tombe en dessous d'une valeur seuil prédéterminée, dans lequel en particulier pour mesurer la turbidité du milieu de stérilisation traité, une extinction et/ou une diffusion d'un rayonnement supplémentaire (58) traversant le milieu de stérilisation traité, en particulier un rayonnement infrarouge, est déterminée.

4. Dispositif de mesure (34) destiné à être utilisé dans une installation de remplissage de boisson (10) et pour déterminer une concentration d'acide peracétique dans un flux de milieu de stérilisation (16) contenant de l'acide peracétique et du peroxyde d'hydrogène circulant à travers l'installation de remplissage de boisson (10), comprenant :
- une unité d'apport (36) pour introduire une enzyme catalase dans le flux de milieu de stérilisation (16 ; 66) circulant à travers l'installation de remplissage de boisson (10) et
- une unité de mesure optique (38) agencée en aval de l'unité d'apport (36) pour déterminer la concentration d'acide peracétique dans le flux de milieu de stérilisation (16 ; 66),
**caractérisé en ce que** le dispositif de mesure comprend
- une unité de mesure de turbidité (54) agencée en aval de l'unité d'apport (36), qui est conçue pour mesurer une turbidité du flux de milieu de stérilisation (16 ; 66), pour déterminer une fin de réaction d'une conversion de peroxyde d'hydrogène dans le flux de milieu de stérilisation en oxygène provoquée par une enzyme catalase et de l'eau (16 ; 66).

5. Dispositif de mesure selon la revendication 4, **caractérisé en ce que** l'unité de mesure optique (38) comprend une source de rayonnement (40) pour émettre un rayonnement (42) traversant le flux de milieux de stérilisation (16 ; 66), en particulier un rayonnement UV, et un capteur de lumière (44) pour mesurer le rayonnement (42) traversant le flux de milieu de stérilisation (16 ; 66).

6. Dispositif de mesure selon la revendication 4 ou 5, dans lequel le dispositif de mesure comprend une section de séjour (46) agencée entre l'unité d'apport (36) et l'unité de mesure optique (38), qui peut être traversée par le flux de milieu de stérilisation (16) depuis l'unité d'apport (36) dans la direction de l'unité optique (38), dans lequel en particulier la section de séjour (46) présente une longueur d'au moins 2 m à 25 m, en particulier une longueur de sensiblement 5 m.

7. Dispositif de mesure selon l'une des revendications 4 à 6, **caractérisé en ce qu'**il comporte une unité de mesure de débit d'écoulement (50) pour déterminer un débit d'écoulement, en particulier un débit massique et/ou volumique, du flux de milieu de stérilisation (16 ; 66) circulant à travers le dispositif de mesure (34).

8. Dispositif de mesure selon l'une des revendications 4 à 7, **caractérisé en ce que** l'unité de mesure de turbidité (54) comprend une source de lumière supplémentaire (56) pour émettre un rayonnement infrarouge (58) transilluminant le flux de milieu de stérilisation et un capteur de lumière supplémentaire (56) pour mesurer le rayonnement infrarouge (58) passant à travers le flux de milieu de stérilisation (16 ; 66), et dans lequel en particulier l'unité de mesure de turbidité (54) est conçue pour déterminer une fin de réaction lorsqu'une valeur mesurée de la turbidité tombe en dessous d'une valeur de seuil prédéterminée.

9. Dispositif de mesure selon la revendication 8, **caractérisé en ce que** l'unité de mesure optique (38) est conçue pour déterminer la concentration d'acide peracétique dans le flux de milieu de stérilisation (16 ; 66) lorsqu'une fin de réaction de la conversion de peroxyde d'hydrogène provoquée par enzyme catalase dans le flux de milieu de stérilisation (16 ; 66) est déterminée par l'unité de mesure de turbidité (54).

10. Dispositif de mesure selon l'une des revendications 4 à 9, dans lequel le dispositif de mesure comprend une unité de commande (52), à laquelle l'unité de mesure optique (38) et/ou l'unité d'apport (36) est/sont connectée(s), qui commande un afflux d'acide peracétique et/ou de l'enzyme catalase dans le flux de milieu de stérilisation (16 ; 66) en fonction de la concentration d'acide peracétique déterminée par l'unité de mesure optique (38).

11. Dispositif de mesure selon la revendication 10, **caractérisé en ce qu'**il comprend une unité de mesure optique supplémentaire (62) agencée en amont de l'unité d'apport (36) pour déterminer une concentration d'acide peracétique et de peroxyde d'hydrogène dans le flux de milieu de stérilisation (16), qui est connectée à l'unité de commande (52), dans lequel l'unité de commande (52) est conçue de sorte qu'en fonction de la concentration d'acide peracétique déterminée par l'unité de mesure optique (38) dans le flux de milieu de stérilisation (16 ; 66) en aval de l'unité d'apport (36) et de la concentration, déterminée par l'unité de mesure optique supplémentaire (62), d'acide peracétique et de peroxyde d'hydrogène dans le flux de milieu de stérilisation (16) en amont de l'unité d'apport (36) pour déterminer une concentration de peroxyde d'hydrogène dans le flux de milieu de stérilisation (16) en amont de l'unité d'apport (36).

12. Installation de remplissage de boisson (10), comprenant un système de nettoyage (12) pour fournir un flux de milieu de stérilisation (16) s'écoulant à travers l'installation de remplissage de boisson et un dispositif de mesure (34) selon l'une des revendications 4 à 11 pour déterminer une concentration d'acide peracétique dans le flux de milieu de stérilisation s'écoulant à travers l'installation de remplissage de boisson (16).

13. Installation de remplissage de boisson selon la revendication 12, **caractérisé en ce que** le dispositif de mesure (34) peut être alimenté par un flux de dérivation de milieu de stérilisation (66) traversant une conduite de dérivation (64) et le dispositif de mesure (34) est conçu pour traiter le flux de dérivation de milieu de stérilisation (66) circulant à travers la conduite de dérivation (64) au moyen de l'unité d'apport (36) et pour l'analyser au moyen de l'unité de mesure optique (38) afin de déterminer la concentration d'acide peracétique dans le flux de milieu de stérilisation (16).
